# EUROPEAN PATENT SPECIFICATION

(11) **EP 3 415 131 B1**
(45) Date of publication and mention of the grant of the patent: **29.07.2020**
(21) Application number: 18175360.9
(22) Date of filing: 31.05.2018
(51) Int. Cl.: A61F 13/49, A61F 13/15, B32B 37/00, B32B 37/06, B32B 37/14, B26D 1/01

(54) **AN APPARATUS AND METHOD FOR PRODUCING AN ELASTIC COMPOSITE TAPE WITH INTERMITTENT ELASTIC SECTIONS**
VORRICHTUNG UND VERFAHREN ZUR HERSTELLUNG EINES ELASTISCHEN VERBUNDBANDES MIT INTERMITTIERENDEN ELASTISCHEN ABSCHNITTEN
APPAREIL ET PROCÉDÉ DE PRODUCTION D'UNE BANDE ÉLASTIQUE COMPOSITE À SECTIONS ÉLASTIQUES INTERMITTENTES

(30) Priority: 13.06.2017 IT 201700065365
(43) Date of publication of application: 19.12.2018
(73) Proprietor: Fameccanica.Data S.p.A., 65129 Pescara (IT)
(72) Inventor: D'APONTE, Francesco, I-65120 Pescara (IT)
(74) Representative: Marchitelli, Mauro

(56) References cited:
- EP-A1- 3 028 687
- WO-A1-2016/187431

## Description

### Field of the invention

The present invention relates to an apparatus and a method for producing an elastic composite tape, with elastic sections spaced apart from each other alternately by a first pitch and by a second pitch, which are different from each other, in the longitudinal direction of the tape.

The present invention has been developed in particular for producing elastic tapes intended to be applied in the waist region of disposable absorbent sanitary articles, for example, of the type for babies or for incontinent adults. However, the scope of the invention is not limited to this possible field of application.

### Description of the prior art

Absorbent sanitary products, of the type for both incontinent adults and babies, are often obtained from two continuous composite tapes spaced apart from each other in a transversal direction, which advance in the longitudinal direction, between which absorbent cores extend, transversely arranged with respect to the longitudinal direction. Composite tapes form the front and back waist regions of the absorbent sanitary products. The waist regions are often equipped with elastic tapes in order to give the absorbent product better adherence to the user's body.

A well-established technique for producing elastic tapes for absorbent sanitary products involves applying a continuous elastic film in a tensioned state between two nonwoven sheets. In the case of absorbent products for adults it is favorable to provide composite elastic tapes in which the elastic film is interrupted, typically at the absorbent core.

US2010/0051170 describes a method for producing an elastic composite tape with intermittent elastic sections. The method described in this document envisages: forming sections of stretched elastic sheets starting from a continuous elastic sheet, feeding a continuous sheet in a longitudinal direction, arranging the elastic sections intermittently on the continuous sheet with the tensioning direction of the elastic sections aligned with the longitudinal direction of the sheet, fixing the opposite ends of the elastic sections to the continuous sheet by means of adhesive, and fixing the elastic sections and the continuous sheet at the intermediate zones between the opposite end portions by means of a spot welding pattern using ultrasonic welding.

EP3028687A1, assigned to the same applicant, describes a method and an apparatus for producing an elastic composite tape with intermittent elastic sections, which only uses welding, typically ultrasonic, to produce the composite tape.

While the method and the apparatus described in EP3028687A1 are advantageous for producing elastic composite tapes intended to be incorporated in the waist regions of a disposable absorbent sanitary article, where the elastic film comprised in the composite tape is typically absent at the absorbent core, they can be further improved by making it possible to produce absorbent articles comprising elastic composite tapes in the waist regions, in which the elastic film is absent not only at the absorbent core, but also in the side zones adjacent to the side edges of the front and back waist regions of the absorbent article, along which the composite tape is typically cut transversely in the production line to form the individual articles.

This characteristic is particularly appreciable in a typical method for manufacturing pre-closed absorbent articles of the training-pant type, in which typically the last step envisages that the composite tapes corresponding to the front and back waist regions to be superimposed on each other, and the individual articles are formed by making the transverse cuts and, at the same time, the side welds. The cut, and particularly the welds, would thus generally involve four layers of material, typically nonwoven material substantially homogeneous to each other, without the presence of intermediate elastic films, which generally comprise materials of different chemical and physical nature with respect to nonwoven materials, thus rendering the production of reliable welds more problematic.

### Object and summary of the invention

The present invention aims to provide an apparatus and a method for producing an elastic composite tape with intermittent elastic sections spaced apart from each other in the longitudinal direction alternately by a first distance and by a second distance, which are different from each other, which do not use adhesives.

According to the present invention, this object is achieved by a method and by an apparatus having the characteristics forming the subject of the attached claims.

The claims form an integral part of the disclosure provided here in relation to the invention.

### Brief description of the drawings

The present invention will now be described in detail with reference to the attached drawings, given purely by way of non-limiting example, wherein:
- Figure 1 is a schematic side view of an apparatus according to the present invention,
- Figure 2A is a schematic plan view illustrating the steps of the method according to the invention,
- Figure 2B is a schematic plan view illustrating the formation of an absorbent sanitary article of the pre-closed type using the elastic composite tape produced according to the invention,
- Figure 3 is a perspective view of a transport unit indicated by the arrow III in Figure 1,
- Figure 4 is an enlarged detail of the part indicated by the arrow IV in Figure 3,
- Figure 5 is a perspective view of an anvil roller indicated by the arrow V in Figure 1,
- Figure 6 is an enlarged detail of the part indicated by the arrow VI in Figure 5,
Figure 7 is a schematic side view of an alternative embodiment of an apparatus according to the invention, and
- Figure 8 is a perspective view of a transport unit indicated by the arrow VII in Figure 7,

### Detailed description

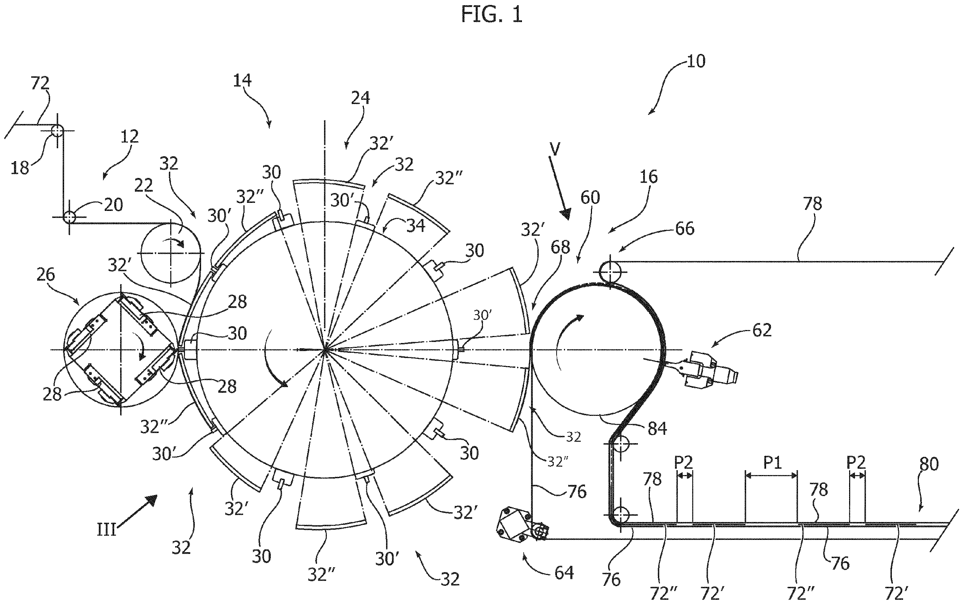

With reference to Figure 1, numeral 10 indicates a first embodiment of an apparatus for forming an elastic composite tape that can be used to produce the waist bands of disposable absorbent sanitary products. The apparatus 10 comprises a feeding and tensioning unit 12, a cutting and pitch-varying unit 14, typically called a cut and repitch unit, and a welding unit 16.

The feeding and tensioning unit 12 comprises a plurality of tensioning rollers 18, 20, 22, which typically can be independently motorized, configured to feed a continuous elastic film 72 in a longitudinal direction and to impart longitudinal tension to the continuous elastic film 72.

The longitudinal tensioning of the continuous elastic film 72 can be obtained, for example, by controlling the peripheral speed of the tensioning rollers 18, 20, 22, so that the downstream tensioning rollers have a peripheral speed greater than the upstream tensioning rollers. Alternatively, the downstream tensioning roller 22 could be motorized and the tensioning rollers 18, 20 further upstream could be braked so as to impart longitudinal tension to the continuous elastic film 72 upstream of the tensioning roller 22. The operation and the control modalities of the tensioning unit 12 are well known in the sector of producing elastic tapes for absorbent sanitary products.

The cut and repitch unit 14 comprises a pitch-varying device, or rotating repitch device 24 and a knife roller 26. The knife roller 26 comprises one or more cutting elements 28, which cooperate with first anvils 30 and second anvils 30' of the rotating repitch device 24, for transversely cutting the continuous elastic film 72 while this is retained in a tensioned state.

The rotating repitch device 24 comprises a plurality of transport units 32, each comprising a first section 32' and a second section 32", carried by a rotating support 34. The first and second anvils 30, 30' are fixed with respect to the rotating support 34. In particular, each first anvil 30 is positioned between two consecutive transport units 32, while each second anvil 30' is positioned between two sections 32' and 32" of a respective transport unit 32. In the illustrated embodiment, the first and second sections 32', 32" of the transport untis 32 are rotatably fixed with respect to the rotating support 34, and are movable in a radial direction between a gripping position and a release position. In the release position, the first and second sections 32', 32" of the transport units 32 are moved radially outwards relative to the gripping position. During rotation, the first and second sections 32', 32" of the transport units 32 move cyclically between the gripping position and the release position. A rotating repitch device of this type is described, for example, in US-A-4,617,082. This type of repitch device is not imperative. Any other type of device could be used capable of spacing apart subsequent discrete elements by a predetermined pitch. For example, a device may be used in which the transport units move along a closed - but not circular - path, as an alternative to devices of the illustrated type rotating about an axis.

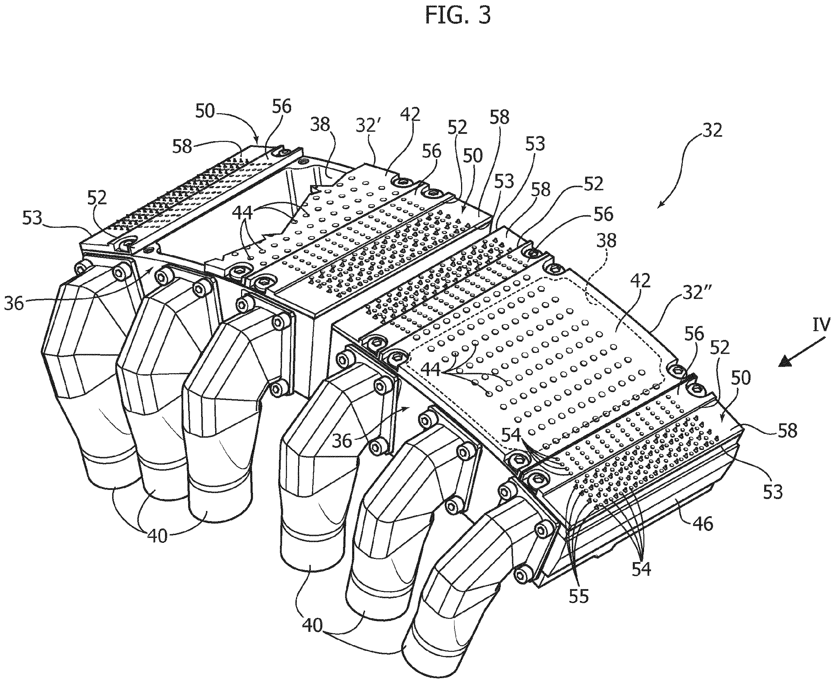

With reference to Figure 3, the first section 32' and the second section 32", typically identical to each other, each comprise a body 36 having a plurality of suction chambers 38 that communicate with a sub-atmospheric pressure source by means of respective ducts 40. The upper part of the suction chambers 38 is closed by a cylindrical surface 42 provided with holes 44.

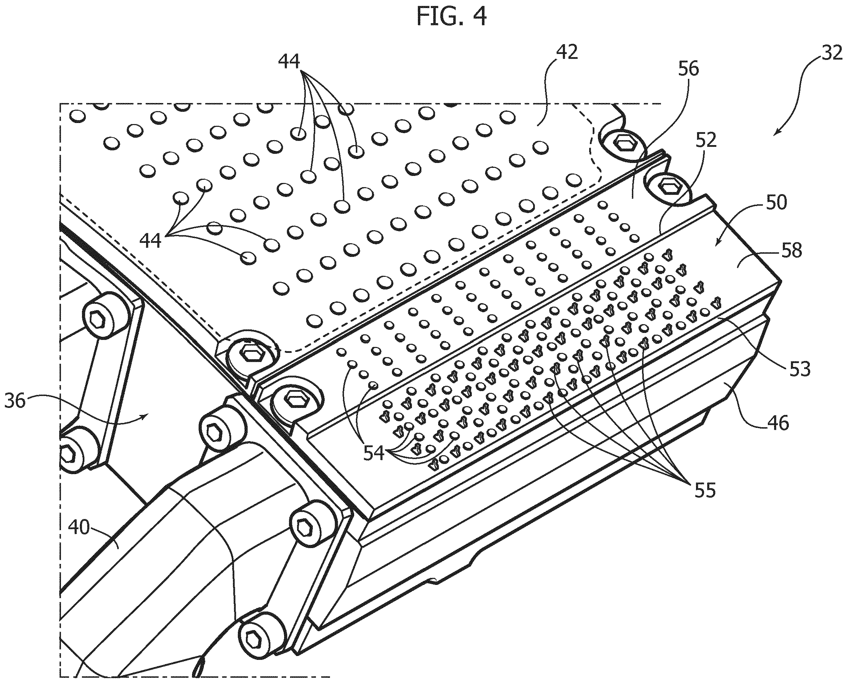

Each transport unit 32 as a whole has substantially the shape of a cylindrical sector with an angular extension equal to the angular distance between two adjacent anvils 30. Likewise, the respective sections 32' and 32" of each unit 32 have an angular extension equal to the angular distance between a first anvil 30 and a second adjacent anvil 30'. The first and second sections 32' and 32" of each transport unit 32 each comprise a hollow body 46 connected to the sub-atmospheric pressure source via a respective duct 40. The body 46 has two specular longitudinal end surfaces 50, each of which, in the embodiment illustrated in Figures 3 and 4, is typically provided with a gripping surface 58 delimited by the transverse groove 52 and the respective longitudinal end edge 53 of each section 32', 32" of the transport unit 32. The transverse groove 52 therefore divides each end surface 50 into an inner portion 56 and a gripping surface 58. Each end surface 50 is provided with holes 54. Preferably, the inner portion 56 of each end surface 50 is smooth, while the gripping surface 58 is provided with protruding teeth 55.

In an alternative embodiment, the end surfaces 50 may comprise only the gripping surfaces 58 provided with holes 54 and protruding teeth 55.

The welding unit 16 comprises an anvil roller 60 with a suction surface and an ultrasonic welding head 62 cooperating with the outer surface of the anvil roller 60.

The apparatus 10 also comprises a first feeding system 64 and a second feeding system 66, for feeding a first web material 76 and a second web material 78 onto the outer surface of the anvil roller 60, respectively. The two web materials 76 and 78 may preferably be continuous sheets of nonwoven material.

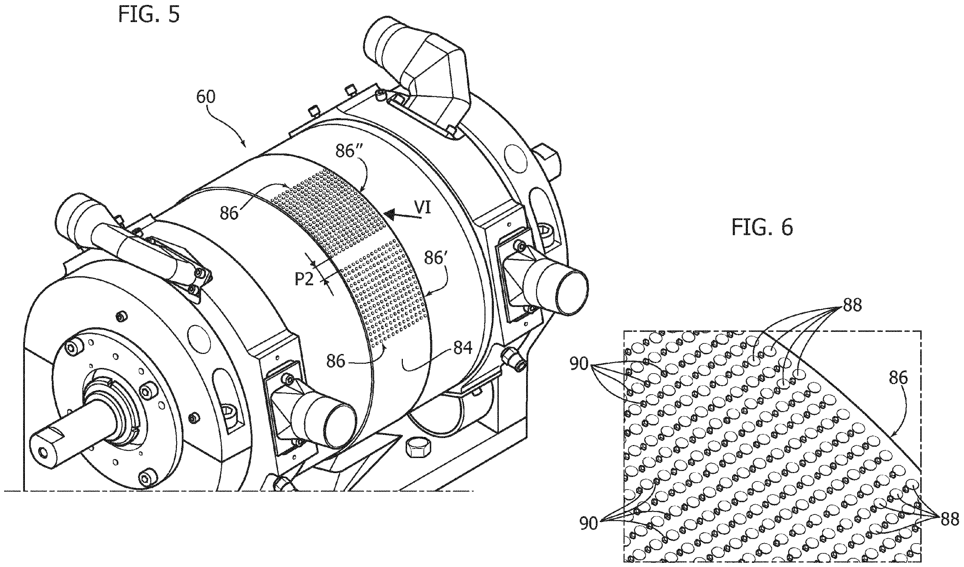

With reference to Figure 5, the anvil roller 60 has a cylindrical contrast surface 84 provided with protruding teeth 90 and one or more suction sections 86. Each suction section 86 is provided with holes 88 that communicate with a suction cavity connected to a sub-atmospheric pressure source. In the embodiment illustrated in Figure 5, the anvil roller 60 has at least one pair of suction sections 86' and 86" spaced apart from each other by a distance equal to the required second distance P2 between successive elastic sections. In the invention several pairs of suction sections 86', 86" are provided, said pairs are spaced apart from each other by a distance equal to the first required distance P1 between successive elastic sections. Each suction section 86 typically has a length equal to the length of a stretched elastic section. The cylindrical contrast surface 84, in the sections included between two consecutive suction sections 86, may be without suction holes 88.

Each suction section 86 of the cylindrical contrast surface 84 is provided with protruding teeth 90 uniformly distributed on the suction section 86. The protruding teeth 90 have head surfaces which form counter-welding surfaces cooperating with the sonotrode of the ultrasonic welding head 62. Preferably, the teeth 90 have a rhomboidal cross-section. The teeth 90 typically cooperate with the holes 88 that are typically intercalated with them. In the illustrated example, the holes 88 and the protruding teeth 90 are aligned in parallel transverse rows. The teeth 90 and the holes 88 are alternated with each other in each transverse row. Preferably, the teeth 90 may be present on the entire outer surface of the anvil roller 60.

The operation of the apparatus 10 is as follows.

The continuous elastic film 72 is subjected to longitudinal tensioning in the feeding and tensioning unit 12, and is fed in a tensioned state to the first section 32' of a transport unit 32 of the repitch device 24. The head portion of the continuous elastic film 72 is retained by suction at the first section 32' of the transport unit 32. The protruding teeth 55 of the gripping surface 58 facilitate the gripping of the head portion of the continuous elastic film 72. The longitudinal tension applied to the continuous elastic film 72 by the tensioning unit 12 is maintained during the transfer of the head portion of the continuous elastic film 72 from the last roller 22 of the tensioning unit 12 to the first section 32' of the transport unit 32.

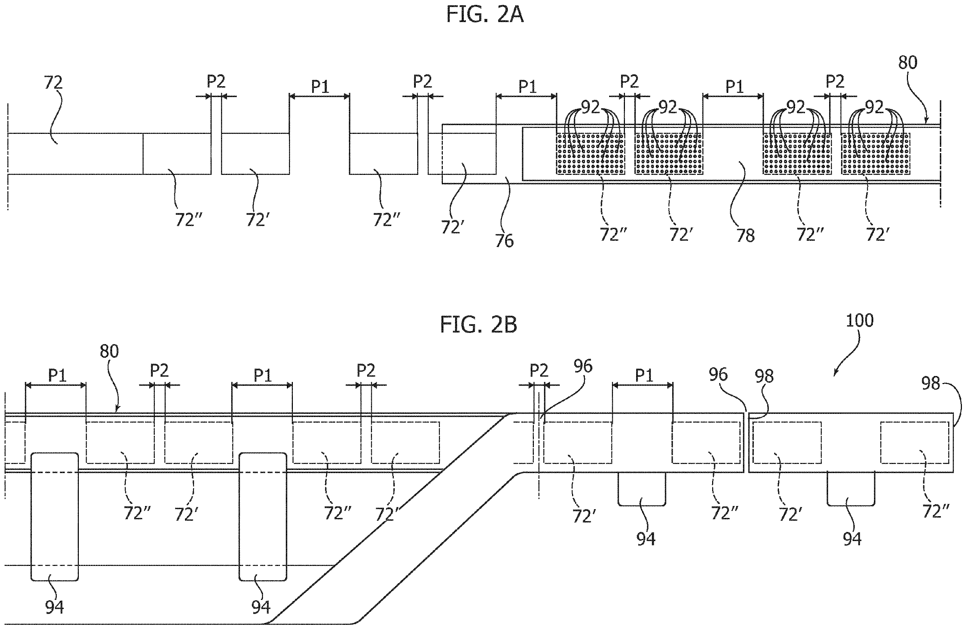

While the head portion of the continuous elastic film 72 is retained in a tensioned state on the surface of the first section 32' and, subsequently, of the second section 32" of the transport unit 32 of the repitch device 24, the knife roller 26 performs the transverse cut of the continuous elastic film 72 on a first anvil 30 and then on the subsequent second anvil 30' of the repitch device 24. In this way, successive discrete sections of elastic film 72' and 72" are formed on the sections 32' and 32" of each transport unit 32 of the repitch device 24. The discrete sections of elastic film 72' and 72" are retained in a tensioned state on the respective sections 32' and 32" of each transport unit 32. During rotation of the repitch device 24, the discrete sections of elastic film 72' and 72" are first spaced apart from each other by a second distance P2 and thereafter each pair of discrete sections of elastic film 72' and 72" is spaced from the subsequent pair by a first distance P1. The first distance P1 is greater than the second distance P2, and generally, in a production line of absorbent sanitary articles of the pre-closed type 100 schematically illustrated in Figure 2B, corresponds to the area in the waist bands where the absorbent core 94 is positioned. The second distance P2 typically corresponds to the areas of the waist bands where the transverse cuts 96 are made and - at the same time - the side welds 98, to form the individual pre-closed absorbent sanitary articles 100. The cut and in particular the weld, produced by known means, are favored by the absence of the elastic film in the two composite materials, which can thus be joined together in an effective and reliable manner.

The first distance P1 is between 5 and 50 mm, in particular between 10 and 20 mm. The second distance P2 typically depends on the size of the absorbent core, and is between 100 and 300 mm, typically between 150 and 250 mm.

The sections 32' and 32" of the transport units 32 in the release position pass through a tangency zone 68, in which the outer surface of each transport unit is tangent to the outer surface 84 of the anvil roller 60. In the tangency zone 68, the discrete sections of elastic film 72' and 72" are transferred from the sections 32' and 32" of each transport unit 32 to the anvil roller 60 of the welding unit 16. The discrete sections of elastic film 72' and 72" are retained in a tensioned manner in the longitudinal direction during transfer from the sections 32' and 32" of the transport unit 32 to the anvil roller 60.

The first continuous web of nonwoven material 76 is fed to the outer surface of the anvil roller 60 upstream of the tangency zone 68 . The first continuous web of nonwoven material 76 is, therefore, arranged between the outer cylindrical surface 84 of the anvil roller 60 and the discrete sections of elastic film 72' and 72". The discrete sections of elastic film 72' and 72" are positioned on respective suction sections 86' and 86", as illustrated in Figure 5, where they are retained by suction. The first continuous web of nonwoven material 76 is porous and, therefore, does not hinder the transfer and retention operations by suction of the discrete sections of elastic film 72' and 72". The protruding teeth 90 arranged on the suction sections 86' and 86" of the anvil roller 60 in cooperating positions, typically intercalated with the suction holes 88, allow the discrete sections of elastic film 72' and 72" to be retained in a tensioned state without the need to apply glue between the discrete sections of elastic film 72' and 72" and the first continuous sheet of nonwoven material 76. On the anvil roller 60, the discrete sections of elastic film 72' and 72" are spaced apart from each other by the second distance P2 equal to the distance between the sections 32' and 32" of each transport unit 32 of the repitch device 24 in the tangency zone 68, while each pair of discrete sections of elastic film 72' and 72" is spaced apart from the next pair by the first distance P1, equal to the distance between each pair of sections 32' and 32" and the next, i.e. equal to the distance between the transport units 32, in the tangency zone 68.

Subsequently, the second continuous web of nonwoven material 78 is fed onto the cylindrical surface 84 of the anvil roller 60 downstream of the tangency zone 68. In this way, the two web materials 76 and 78 include the discrete sections of elastic film 72' and 72" between them.

Downstream of the feeding zone of the second continuous nonwoven web 78 at the anvil roller 60, the welding head 62 carries out the welding of the first continuous sheet 76, of the discrete sections of elastic film 72' and 72" and of the second continuous nonwoven web 78 on the head surfaces of the protruding teeth 90.

In the areas in which the discrete elastic film sections 72' and 72" are spaced apart from each other, the welding head 62 directly welds the first continuous nonwoven web 76 and the second continuous nonwoven web 78 on the head surfaces of the protruding teeth 90, in the case in which the teeth 90 are preferably present, for example, on the entire outer surface of the anvil roller 60.

The product obtained downstream of the welding head 62 is a continuous composite tape 80 with discrete sections of elastic film 72' and 72" tensioned in the longitudinal direction and spaced apart alternately by a first predetermined distance P1 and by a second distance P2, as illustrated in Figure 2A. The discrete sections of elastic film 72' and 72" are sandwiched between the two continuous nonwoven webs 76, 78. A particularly important characteristic of the present invention is that the discrete sections of elastic film 72' and 72" are fixed to the two continuous sheets 76, 78 exclusively by welding, without using any glue. With the solution according to the present invention it is possible to avoid the use of glue because the discrete sections of elastic film 72' and 72" are applied directly on suction sections 86' and 86" of the anvil roller 60 provided with protruding teeth 90 that form the counter-welding surfaces on which the welding is performed, with reference to the anvil roller 60 illustrated, for example, in Figure 5. The welding spots 92 formed by the protruding teeth 90 on the continuous composite tape 80 are indicated in Figure 2A only at the discrete sections of elastic film 72' and 72", consistently with the configuration of the anvil roller 60 illustrated in Figure 5. Alternatively, the continuous composite tape 80 may have the welding spots on the entire surface, in the case in which the anvil roller 60 preferably comprises protruding teeth 90 on the entire outer surface.

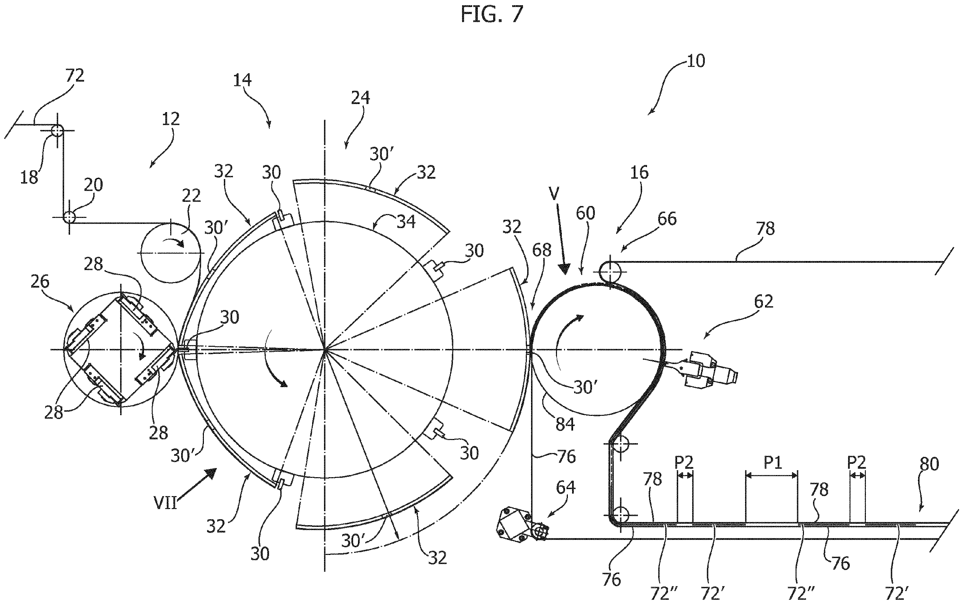

Figure 7 illustrates a further embodiment of a cut and repitch unit 14 according to the present invention, which differs from that illustrated in Figure 1 in its configuration of the rotating repitch device 24 with the respective transport units 32, the other components being substantially equivalent.

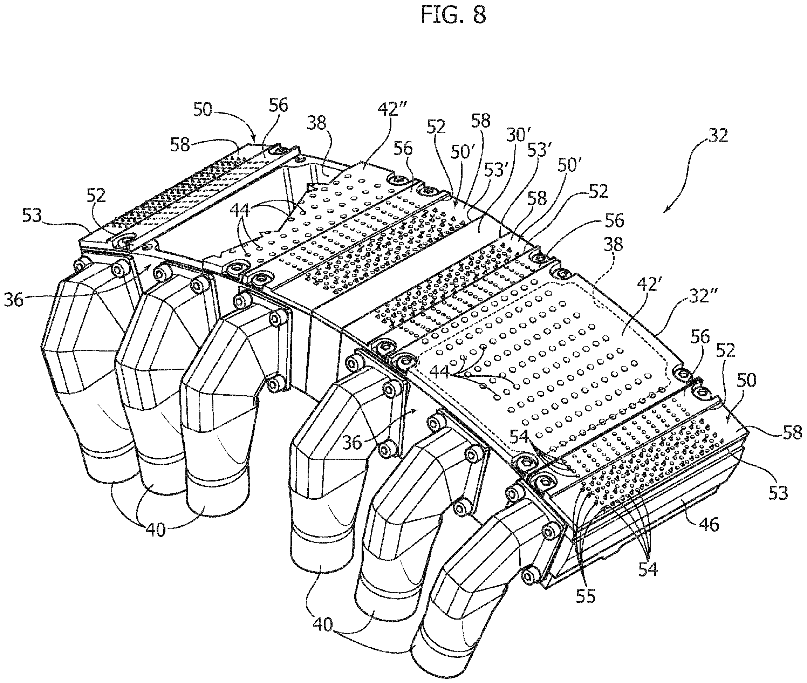

The rotating repitch device 24 comprises a plurality of transport units 32, illustrated in greater detail in Figure 8, carried by a rotating support 34. Also in this embodiment, the transport units 32 are rotatably fixed with respect to the rotating support 34 and are movable in a radial direction between a gripping position and a release position, in which they are moved radially outwards with respect to the gripping position. During rotation, the gripping units 32 move cyclically between the gripping position and the release position. Of course, other known types of repitch devices could be used to space apart successive discrete elements from each other in the required manner.

The rotating repitch device 24 comprises a plurality of anvils 30 fixed with respect to the rotating support 34.

With reference to Figure 8, each transport unit 32 comprises a body 36 having a plurality of suction chambers 38 that communicate with a sub-atmospheric pressure source via respective ducts 40. The upper part of the suction chambers 38 is closed by cylindrical surfaces 42', 42" provided with holes 44. Each transport unit 32 has substantially the shape of a cylindrical sector with an angular extension equal to the angular distance between two adjacent anvils 30. Each transport unit 32 also comprises an intermediate anvil 30' typically located between the cylindrical surfaces 42' and 42", and without suction holes. The body 46 has two specular longitudinal end surfaces 50, and two intermediate surfaces 50' symmetrically located on the two sides of the intermediate anvil 30'. The two end surfaces 50 and the two intermediate surfaces 50', in the embodiment illustrated in Figure 8, are typically provided with a gripping surface 58 delimited in a longitudinal direction by the transverse groove 52 and, respectively, by a longitudinal end edge 53 of the transport unit 32, and by the respective transverse edge 53' of the intermediate anvil 30'. The transverse groove 52 therefore divides each end surface 50 and intermediate surface 50' into an inner portion 56 and a gripping surface 58. Each end 50 and intermediate surface 50' is provided with holes 54; preferably, the inner portion 56 of each end surface 50 and intermediate surface 50' is smooth, whereas the gripping surface 58 is provided with protruding teeth 55.

Also in this embodiment, the end surfaces 50 and the intermediate surfaces 50' may comprise only the gripping surfaces 58 provided with holes 54 and protruding teeth 55.

During operation of the rotating repitch device 24 in the alternative embodiment illustrated in Figures 7 and 8, the continuous elastic film 72 is subjected to longitudinal tensioning in the feeding and tensioning unit 12 and is fed in a tensioned state to a transport unit 32 of the repitch device 24. The head portion of the continuous elastic film 72 is retained by suction at the transport unit 32. The protruding teeth 55 of the gripping surface 58 facilitate the gripping of the head portion of the continuous elastic film 72. The longitudinal tension applied to the continuous elastic film 72 by the tensioning unit 12 is maintained during transfer of the head portion of the continuous elastic film 72 from the last roller 22 of the tensioning unit 12 to the transport unit 32.

While the head portion of the continuous elastic film 72 is retained in a tensioned state on the surface of the transport unit 32 of the repitch device 24, the knife roller 26 performs the transverse cut of the continuous elastic film 72 on an anvil 30 of the repitch device 24, and then on the subsequent intermediate anvil 30' of the transport unit 32. In this way, successive discrete sections of elastic film 72' and 72" are formed on the transport unit 32 of the repitch device 24. The discrete sections of elastic film 72' and 72" are held in a tensioned state on the gripping surfaces 58 of the two end surfaces 50 and of the two intermediate surfaces 50' of each respective transport unit 32. At the moment of cutting on the intermediate anvil 30', the adjacent portions of the film positioned at the intermediate anvil 30' itself partially withdraw due to the absence of suction holes, producing the distancing of the discrete sections of elastic film 72' and 72" formed on each transport element 32 by the predetermined distance equal to the second distance P2. Subsequently, each pair of discrete sections of elastic film 72' and 72" on each transport element 32 is spaced apart from the next pair on the subsequent transport element 32 by a first distance P1, with the first distance P1 greater than the second distance P2.

The alternative embodiment of the rotating repitch device 24 illustrated in Figure 7 presents, with respect to the embodiment illustrated in Figures 1, 3 and 4, a greater constructive and operating simplicity, having, on the other hand, some limited portions of the elastic film 72 that do not participate in the elasticization of the resulting composite material, in particular those that withdraw in the untensioned state after cutting at the intermediate anvil 30'.

Of course, without prejudice to the principle of the invention, the details of construction and the embodiments may be widely varied with respect to those described and illustrated, without thereby departing from the scope of the invention as defined by the claims that follow.

## Claims

1. An apparatus for producing an elastic composite tape (80) with intermittent elastic sections (72', 72"), comprising:
- a tensioning unit (12) for longitudinally tensioning a continuous elastic film (72),
- a cut and repitch unit (14) comprising a knife roller (26) and a repitch device (24) comprising a plurality of transport units (32) and a plurality of anvils (30, 30') cooperating with the knife roller (26) to transversely cut the tensioned continuous elastic film (72) and to form a succession of discrete sections of elastic film (72', 72"), wherein the transport units (32) retain - by suction - respective discrete sections of elastic film (72', 72") alternately spaced apart from each other by a first predetermined distance (P1) and by a second predetermined distance (P2), with said first distance (P1) greater than said second distance (P2), wherein the first distance (P1) is comprised between 5 and 50 mm, in particular between 10 and 20 mm, and wherein the second distance (P2) is comprised between 100 and 300 mm, in particular between 150 and 250 mm, wherein each of said transport units (32) comprises a first section (32') and a second section (32"), wherein said transport units (32) retain said discrete sections of elastic film (72', 72") spaced apart from each other by said first distance (P1) and said first section (32') and second section (32") of each transport unit (32) retain said discrete sections of elastic film (72', 72") spaced apart from each other by said second distance (P2),
- a welding unit (16) comprising an anvil roller (60) and a welding head (62) cooperating with the anvil roller (60), wherein the anvil roller (60) has a cylindrical contrast surface (84) provided with a plurality of protruding teeth (90) having head surfaces forming counter-welding surfaces that cooperate with said welding head (62), wherein the anvil roller (60) has a plurality of pairs of suction sections (86', 86") provided with holes (88) that communicate with a suction cavity connected to a sub-atmospheric pressure source, wherein each suction section (86', 86") has a length equal to the length of the stretched elastic sections (72', 72"), wherein the suction sections (86', 86") of each pair are spaced apart from each other by a distance equal to said second distance (P2) between successive elastic sections (72', 72"), and wherein said pairs of suction sections (86', 86") are spaced apart from each other by a distance equal to the first required distance (P1) between successive elastic sections (72', 72"),
- a tangency zone (68) between the transport units (32) and the anvil roller (60), in which respective discrete sections of elastic film (72', 72") are transferred to said at least one suction section (86) of the anvil roller (60),
- a first feeding system (64) for feeding a first web material (76) to the anvil roller (60) upstream of said tangency zone (68), and
- a second feeding system (66) for feeding a second web material (78) to the anvil roller (60) between said tangency zone (68) and said welding head (62) .

2. An apparatus according to claim 1, wherein each of said transport units (32) comprises an intermediate anvil (30') cooperating with said knife roller (26) to form two discrete sections of elastic film (72', 72'), wherein said transport units (32) retain said discrete sections of elastic films (72') spaced apart from each other by said first distance (P1), and each transport unit (32) at least partially retains - by suction - said two discrete sections of elastic film (72', 72") spaced apart from each other by said second distance (P2) .

3. An apparatus according to claim 1 or claim 2, wherein said protruding teeth (90) have a rhomboidal cross-section.

4. An apparatus according to any claim 1 or claim 2, wherein said suction holes (88) and said teeth (90) are arranged in transverse rows, wherein in each transverse row, the suction holes (88) and said protruding teeth (90) are alternated with each other.

5. A method for producing an elastic composite tape (80) with intermittent elastic sections (72', 72"), comprising:
- tensioning a continuous elastic film (72) in a longitudinal direction,
- cutting the continuous elastic film (72) in a transverse direction to form successive sections of elastic film (72', 72") tensioned in the longitudinal direction,
- spacing said discrete sections of elastic film (72', 72") alternately by a first predetermined distance (P1) and by a second predetermined distance (P2), with said first distance (P1) greater than said second distance (P2), wherein the first distance (P1) is comprised between 5 and 50 mm, in particular between 10 and 20 mm, and wherein the second distance (P2) is comprised between 100 and 300 mm, in particular between 150 and 250 mm, wherein each of said transport units (32) comprises a first section (32') and a second section (32"), wherein said transport units (32) retain said discrete sections of elastic film (72', 72") spaced apart from each other by said first distance (P1) and said first section (32') and second section (32") of each transport unit (32) retain said discrete sections of elastic film (72', 72") spaced apart from each other by said second distance (P2),
- transferring said discrete sections of tensioned elastic film (72', 72") onto an anvil roller (60) of a welding unit (16) having a cylindrical contrast surface (84) provided with a plurality of protruding teeth (90) having head surfaces forming counter-welding surfaces, wherein the anvil roller (60) has a plurality of pairs of suction sections (86', 86") provided with holes (88) that communicate with a suction cavity connected to a sub-atmospheric pressure source, wherein each suction section (86', 86") has a length equal to the length of the stretched elastic sections (72', 72"), wherein the suction sections (86', 86") of each pair are spaced apart from each other by a distance equal to said second distance (P2) between successive elastic sections (72', 72"), and wherein said pairs of suction sections (86', 86") are spaced apart from each other by a distance equal to the first required distance (P1) between successive elastic sections (72', 72"),
- feeding a first and a second continuous web material (76, 78) to the anvil roller (60),
- enclosing said discrete sections of elastic film (72', 72") between said first and second continuous web material (76, 78), and
- welding to each other said first continuous web material (76), said discrete sections of tensioned elastic film (72', 72") and said second continuous web material (78) on said head surfaces of said protruding teeth (90).

6. A method according to claim 5, wherein at least one of said first and second continuous web materials (76, 78) is a nonwoven web.

## Patentansprüche

1. Vorrichtung zum Herstellen eines elastischen Verbundbands (80) mit intermittierenden elastischen Abschnitten (72', 72"), umfassend:
- eine Spanneinheit (12) zum in Längsrichtung erfolgenden Spannen eines elastischen Endlosfilms (72),
- eine Schneide- und Neuanordnungseinheit (14), die eine Messerwalze (26) und eine Neuanordnungseinrichtung (24) umfasst, die eine Vielzahl von Transporteinheiten (32) und eine Vielzahl von Gegenhaltern (30, 30') umfasst, die mit der Messerwalze (26) zusammenwirken, um den gespannten elastischen Endlosfilm (72) in Querrichtung zu schneiden und eine Abfolge einzelner Abschnitte aus elastischem Film (72', 72") zu bilden, wobei die Transporteinheiten (32) mittels Saugwirkung jeweilige einzelne Abschnitte aus elastischem Film (72', 72") zueinander abwechselnd um einen ersten vorab bestimmten Abstand (P1) und um einen zweiten vorab bestimmten Abstand (P2) beabstandet festhalten, wobei der erste Abstand (P1) größer ist als der zweite Abstand (P2), wobei der erste Abstand (P1) zwischen 5 und 50 mm und insbesondere zwischen 10 und 20 mm umfasst und wobei der zweite Abstand (P2) zwischen 100 und 300 mm und insbesondere zwischen 150 und 250 mm umfasst, wobei jede der Transporteinheiten (32) einen ersten Abschnitt (32') und einen zweiten Abschnitt (32") umfasst, wobei die Transporteinheiten (32) die einzelnen Abschnitte aus elastischem Film (72', 72") zueinander um den ersten Abstand (P1) beabstandet festhalten und der erste Abschnitt (32') und der zweite Abschnitt (32") jeder Transporteinheit (32) die einzelnen Abschnitte aus elastischem Film (72', 72") zueinander um den zweiten Abstand (P2) beabstandet festhalten,
- eine Schweißeinheit (16), die eine Gegenhalterwalze (60) und einen mit der Gegenhalterwalze (60) zusammenwirkenden Schweißkopf (62) umfasst, wobei die Gegenhalterwalze (60) eine zylindrische Gegenfläche (84) aufweist, die mit einer Vielzahl abstehender Zähne (90) versehen ist, die Kopfflächen aufweisen, die Schweißgegenflächen bilden, die mit dem Schweißkopf (62) zusammenwirken, wobei die Gegenhalterwalze (60) eine Vielzahl von Paaren von Ansaugabschnitten (86', 86") aufweist, die mit Löchern (88) versehen sind, die mit einem mit einer Unterdruckversorgung verbundenen Ansaughohlraum in Verbindung stehen, wobei jeder Ansaugabschnitt (86', 86") eine Länge aufweist, die der Länge der gedehnten elastischen Abschnitte (72', 72") gleicht, wobei die Ansaugabschnitte (86', 86") jedes Paars zueinander um einen Abstand beabstandet sind, der dem zweiten Abstand (P2) zwischen aufeinanderfolgenden elastischen Abschnitten (72', 72") gleicht, und wobei die Paare von Ansaugabschnitten (86', 86") zueinander um einen Abstand beabstandet sind, der dem ersten erforderlichen Abstand (P1) zwischen aufeinanderfolgenden elastischen Abschnitten (72', 72") gleicht,
- eine Berührungszone (68) zwischen den Transporteinheiten (32) und der Gegenhalterwalze (60), in der jeweilige einzelne Abschnitte aus elastischem Film (72', 72") an den mindestens einen Ansaugabschnitt (86) der Gegenhalterwalze (60) übergeben werden,
- ein erstes Zuführsystem (64), um der Berührungszone (68) vorgelagert der Gegenhalterwalze (60) ein erstes Bahnmaterial (76) zuzuführen, und
- ein zweites Zuführsystem (66), um der Gegenhalterwalze (60) zwischen der Berührungszone (68) und dem Schweißkopf (62) ein zweites Bahnmaterial (78) zuzuführen.

2. Vorrichtung nach Anspruch 1, wobei jede der Transporteinheiten (32) einen zwischengelagerten Gegenhalter (30') umfasst, der mit der Messerwalze (26) zusammenwirkt, um zwei einzelne Abschnitte aus elastischem Film (72', 72') zu bilden, wobei die Transporteinheiten (32) die einzelnen Abschnitte aus elastischem Film (72') zueinander um den ersten Abstand (P1) beabstandet festhalten und jede Transporteinheit (32) mittels Saugwirkung die zwei einzelnen Abschnitte aus elastischem Film (72', 72") zueinander um den zweiten Abstand (P2) beabstandet zumindest teilweise festhält.

3. Vorrichtung nach Anspruch 1 oder Anspruch 2, wobei die abstehenden Zähne (90) einen rautenförmigen Querschnitt aufweisen.

4. Vorrichtung nach Anspruch 1 oder Anspruch 2, wobei die Ansauglöcher (88) und die Zähne (90) in Querreihen angeordnet sind, wobei in jeder Querreihe die Ansauglöcher (88) und die abstehenden Zähne (90) einander abwechseln.

5. Verfahren zum Herstellen eines elastischen Verbundbands (80) mit intermittierenden elastischen Abschnitten (72', 72"), umfassend:
- Spannen eines elastischen Endlosfilms (72) in einer Längsrichtung,
- Schneiden des elastischen Endlosfilms (72) in einer Querrichtung, um in der Längsrichtung gespannte aufeinanderfolgende Abschnitte aus elastischem Film (72', 72") zu bilden,
- Beabstanden der einzelnen Abschnitte aus elastischem Film (72', 72") abwechselnd um einen ersten vorab bestimmten Abstand (P1) und um einen zweiten vorab bestimmten Abstand (P2), wobei der erste Abstand (P1) größer ist als der zweite Abstand (P2), wobei der erste Abstand (P1) zwischen 5 und 50 mm und insbesondere zwischen 10 und 20 mm umfasst und wobei der zweite Abstand (P2) zwischen 100 und 300 mm und insbesondere zwischen 150 und 250 mm umfasst, wobei jede der Transporteinheiten (32) einen ersten Abschnitt (32') und einen zweiten Abschnitt (32") umfasst, wobei die Transporteinheiten (32) die einzelnen Abschnitte aus elastischem Film (72', 72") zueinander um den ersten Abstand (P1) beabstandet festhalten und der erste Abschnitt (32') und der zweite Abschnitt (32") jeder Transporteinheit (32) die einzelnen Abschnitte aus elastischem Film (72', 72") zueinander um den zweiten Abstand (P2) beabstandet festhalten,
- Übergeben der einzelnen Abschnitte aus gespanntem elastischem Film (72', 72") an eine Gegenhalterwalze (60) einer Schweißeinheit (16), die eine zylindrische Gegenfläche (84) aufweist, die mit einer Vielzahl abstehender Zähne (90) versehen ist, die Schweißgegenflächen bildende Kopfflächen aufweisen, wobei die Gegenhalterwalze (60) eine Vielzahl von Paaren von Ansaugabschnitten (86', 86") aufweist, die mit Löchern (88) versehen sind, die mit einem mit einer Unterdruckversorgung verbundenen Ansaughohlraum in Verbindung stehen, wobei jeder Ansaugabschnitt (86', 86") eine Länge aufweist, die der Länge der gedehnten elastischen Abschnitte (72', 72") gleicht, wobei die Ansaugabschnitte (86', 86") jedes Paars zueinander um einen Abstand beabstandet sind, der dem zweiten Abstand (P2) zwischen aufeinanderfolgenden elastischen Abschnitten (72', 72") gleicht, und wobei die Paare von Ansaugabschnitten (86', 86") zueinander um einen Abstand beabstandet sind, der dem ersten erforderlichen Abstand (P1) zwischen aufeinanderfolgenden elastischen Abschnitten (72', 72") gleicht,
- Zuführen eines ersten und eines zweiten Endlosbahnmaterials (76, 78) an die Gegenhalterwalze (60),
- Einschließen der einzelnen Abschnitte aus elastischem Film (72', 72") zwischen dem ersten und dem zweiten Endlosbahnmaterial (76, 78) und
- Zusammenschweißen des ersten Endlosbahnmaterials (76), der einzelnen Abschnitte aus gespanntem elastischem Film (72', 72") und des zweiten Endlosbahnmaterials (78) auf den Kopfflächen der abstehenden Zähne (90).

6. Verfahren nach Anspruch 5, wobei es sich bei mindestens einem des ersten und des zweiten Endlosbahnmaterials (76, 78) um eine Vliesstoffbahn handelt.

## Revendications

1. Appareil de production d'un ruban composite élastique (80) à sections élastiques intermittentes (72', 72"), comprenant :
- une unité de tension (12) pour tendre longitudinalement un film élastique continu (72),
- une unité de découpe et de redéfinition de pas (14) comprenant un rouleau couteau (26) et un dispositif de redéfinition de pas (24) comprenant une pluralité d'unités de transport (32) et une pluralité d'enclumes (30, 30') coopérant avec le rouleau couteau (26) pour découper transversalement le film élastique continu tendu (72) et pour former une succession de sections discrètes de film élastique (72', 72"), où les unités de transport (32) retiennent - par aspiration - des sections discrètes respectives de film élastique (72', 72") espacées en alternance les unes des autres d'une première distance prédéterminée (P1) et d'une deuxième distance prédéterminée (P2), ladite première distance (P1) étant supérieure à ladite deuxième distance (P2), où la première distance (P1) est comprise entre 5 et 50 mm, en particulier entre 10 et 20 mm, et où la deuxième distance (P2) est comprise entre 100 et 300 mm, en particulier entre 150 et 250 mm, où chacune desdites unités de transport (32) comprend une première section (32') et une deuxième section (32"), où lesdites unités de transport (32) retiennent lesdites sections discrètes de film élastique (72', 72") espacées les unes des autres de ladite première distance (P1) et ladite première section (32') et ladite deuxième section (32") de chaque unité de transport (32) retiennent lesdites sections discrètes de film élastique (72', 72") espacées l'une de l'autre de ladite deuxième distance (P2),
- une unité de soudage (16) comprenant un rouleau d'enclume (60) et une tête de soudage (62) coopérant avec le rouleau d'enclume (60), où le rouleau d'enclume (60) a une surface de contraste cylindrique (84) munie d'une pluralité de dents saillantes (90) ayant des surfaces de tête formant des surfaces de contre-soudage qui coopèrent avec ladite tête de soudage (62), où le rouleau d'enclume (60) a une pluralité de paires de sections d'aspiration (86', 86") munies de trous (88) qui communiquent avec une cavité d'aspiration reliée à une source de pression sub-atmosphérique, où chaque section d'aspiration (86', 86") a une longueur égale à la longueur des sections élastiques étirées (72', 72"), où les sections d'aspiration (86', 86") de chaque paire sont espacées l'une de l'autre d'une distance égale à ladite deuxième distance (P2) entre des sections élastiques successives (72', 72"), et où lesdites paires de sections d'aspiration (86', 86") sont espacées les unes des autres d'une distance égale à la première distance requise (P1) entre les sections élastiques successives (72', 72"),
- une zone de tangence (68) entre les unités de transport (32) et le rouleau d'enclume (60), où des sections discrètes respectives de film élastique (72', 72") sont transférées vers ladite au moins une section d'aspiration (86) du rouleau d'enclume (60),
- un premier système d'acheminement (64) pour acheminer un premier matériau en bande (76) au rouleau d'enclume (60) en amont de ladite zone de tangence (68), et
- un deuxième système d'acheminement (66) pour acheminer un deuxième matériau en bande (78) au rouleau d'enclume (60) entre ladite zone de tangence (68) et ladite tête de soudage (62).

2. Appareil selon la revendication 1, dans lequel chacune desdites unités de transport (32) comprend une enclume intermédiaire (30') coopérant avec ledit rouleau couteau (26) pour former deux sections discrètes de film élastique (72', 72'), où lesdites unités de transport (32) retiennent lesdites sections discrètes de films élastiques (72') espacées les unes des autres de ladite première distance (P1), et chaque unité de transport (32) retient au moins partiellement - par aspiration - lesdites deux sections discrètes de film élastique (72', 72") espacées l'une de l'autre de ladite deuxième distance (P2).

3. Appareil selon la revendication 1 ou 2, dans lequel lesdites dents saillantes (90) ont une section transversale rhomboïde.

4. Appareil selon l'une des revendications 1 et 2, dans lequel lesdits trous d'aspiration (88) et lesdites dents (90) sont agencés en rangées transversales, où, dans chaque rangée transversale, les trous d'aspiration (88) et lesdites dents saillantes (90) sont alternés.

5. Procédé de production d'un ruban composite élastique (80) à sections élastiques intermittentes (72', 72"), comprenant le fait :
- de tendre un film élastique continu (72) dans une direction longitudinale,
- de découper le film élastique continu (72) dans une direction transversale pour former des sections successives de film élastique (72', 72") tendu dans la direction longitudinale,
- d'espacer lesdites sections discrètes de film élastique (72', 72") en alternance d'une première distance prédéterminée (P1) et d'une deuxième distance prédéterminée (P2), ladite première distance (P1) étant supérieure à ladite deuxième distance (P2), où la première distance (P1) est comprise entre 5 et 50 mm, en particulier entre 10 et 20 mm, et où la deuxième distance (P2) est comprise entre 100 et 300 mm, en particulier entre 150 et 250 mm, où chacune desdites unités de transport (32) comprend une première section (32') et une deuxième section (32"), où lesdites unités de transport (32) retiennent lesdites sections discrètes de film élastique (72', 72") espacées les unes des autres de ladite première distance (P1) et ladite première section (32') et ladite deuxième section (32") de chaque unité de transport (32) retiennent lesdites sections discrètes de film élastique (72', 72") espacées l'une de l'autre de ladite deuxième distance (P2),
- de transférer lesdites sections discrètes de film élastique tendu (72', 72") sur un rouleau d'enclume (60) d'une unité de soudage (16) ayant une surface de contraste cylindrique (84) munie d'une pluralité de dents saillantes (90) ayant des surfaces de tête formant des surfaces de contre-soudage, où le rouleau d'enclume (60) a une pluralité de paires de sections d'aspiration (86', 86") munies de trous (88) qui communiquent avec une cavité d'aspiration reliée à une source de pression sub-atmosphérique, où chaque section d'aspiration (86', 86") a une longueur égale à la longueur des sections élastiques étirées (72', 72"), où les sections d'aspiration (86', 86") de chaque paire sont espacées l'une de l'autre d'une distance égale à ladite deuxième distance (P2) entre des sections élastiques successives (72', 72"), et où lesdites paires de sections d'aspiration (86', 86") sont espacées les unes des autres d'une distance égale à la première distance requise (P1) entre les sections élastiques successives (72', 72"),
- d'acheminer des premier et deuxième matériaux en bande continue (76, 78) au rouleau d'enclume (60),
- d'enfermer lesdites sections discrètes de film élastique (72', 72") entre lesdits premier et deuxième matériaux en bande continue (76, 78), et
- de souder ledit premier matériau en bande continue (76), lesdites sections discrètes de film élastique tendu (72', 72") et ledit deuxième matériau en bande continue (78) les uns aux autres sur lesdites surfaces de tête desdites dents saillantes (90).

6. Procédé selon la revendication 5, dans lequel au moins l'un desdits premier et deuxième matériaux en bande continue (76, 78) est une bande non tissée.
